(19) Europäisches Patentamt
European Patent Office
Office européen des brevets

(11) Publication number: **0 598 302 A1**

(12) **EUROPEAN PATENT APPLICATION**

(21) Application number: **93118081.4**

(22) Date of filing: **08.11.93**

(51) Int. Cl.[5]: **C08G 59/30**, C08G 59/62, H01L 23/29, C07D 303/23

(30) Priority: **10.11.92 JP 299702/92**

(43) Date of publication of application: **25.05.94 Bulletin 94/21**

(84) Designated Contracting States: **DE GB**

(71) Applicant: **SUMITOMO CHEMICAL COMPANY, LIMITED**
**5-33, Kitahama 4-chome**
**Chuo-ku**
**Osaka(JP)**

(72) Inventor: **Sugiyama, Yasuhide**
**2-40-1-317, Kasuga**
**Tsukuba-shi, Ibaraki 305(JP)**
Inventor: **Shiomi, Yutaka**
**3-18-5-101, Matushiro**
**Tsukuba-shi, Ibaraki 305(JP)**
Inventor: **Morimoto, Takashi**
**2-40-1-209, Kasuga**
**Tsukuba-shi, Ibaraki 305(JP)**
Inventor: **Saito, Noriaki**
**2-13-1(5-102), Umezono**
**Tsukuba-shi, Ibaraki 305(JP)**
Inventor: **Kitayama, Shinichiro**
**2-13-1(2-103), Umezono**
**Tsukuba-shi, Ibaraki 305(JP)**
Inventor: **Suzuki, Michio**
**680-1, Kawaticho**
**Kasai-shi, Hyogo 666(JP)**
Inventor: **Sakiyama, Kazuo**
**5-20-2, Yamatedai**
**Ibaraki-shi, Osaka 567(JP)**

(74) Representative: **VOSSIUS & PARTNER**
**Siebertstrasse 4**
**D-81675 München (DE)**

(54) **Epoxy resin compositions and resin-encapsulated semiconductor devices.**

(57) An epoxy resin composition which can provide cured products high in adhesion and crack resistance in soldering with high heat resistance and low moisture absorption and a semiconductor devices encapsulated with said composition are disclosed.

The above epoxy resin composition comprises as essential components an epoxy resin represented by the following formula (1):

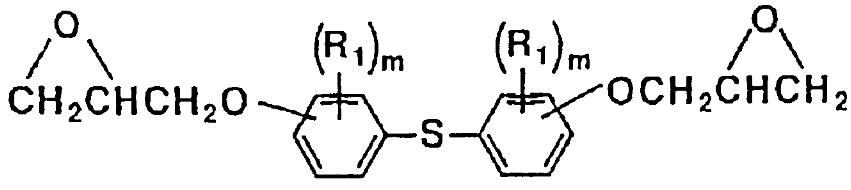

(1)

wherein $R_1$ represents a halogen atom, an alkyl or cycloalkyl group of 1 to 6 carbon atoms, a substituted or unsubstituted phenyl group or a halogen atom and when two or more $R_1$ are present in the same or different rings, they may be the same or different and m represents an integer of 0 to 4, and a polyhydric phenol as a curing agent.

EP 0 598 302 A1

The present invention relates to epoxy resin compositions useful for encapsulating electronic parts and semiconductor devices encapsulated using the compositions.

Recently, semiconductors such as LSI, IC and transistors are encapsulated by transfer molding of economically useful epoxy resin compositions.

Especially, recently LSI is surface mounted and in many cases, LSI is directly dipped in a soldering bath. In this case, since the encapsulant is exposed to high temperatures of higher than 200°C, water absorbed in the encapsulant expands to cause generation of cracks or peeling of the encapsulant at the interface between the encapsulant and the die pad.

Accordingly, epoxy resin encapsulants are demanded to have low moisture absorption and to be improved in crack resistance and adhesion. At present, encapsulants comprising glycidyl ether of o-cresol novolak as an epoxy resin and phenolic novolak as a curing agent are mainly used. However, if they absorb water during storage, the above problems are encountered and they are moistureproof-packed in practical use for avoiding the above problems.

Encapsulants mainly composed of glycidyl ether of o-cresol novolak are somehow balanced in heat resistance and low moisture absorption, but these are not necessarily sufficient in the use which requires high crack resistance in soldering as mentioned above.

The inventors have conducted intensive research on epoxy resin compositions which have high adhesion as well as high heat resistance and low moisture absorption and which can provide cured products excellent in crack resistance in soldering and as a result, have found that a specific epoxy resin composition meets the above objects. Thus, the present invention has been accomplished.

That is, the present invention relates to an epoxy resin composition comprising as essential components an epoxy resin represented by the following formula (1):

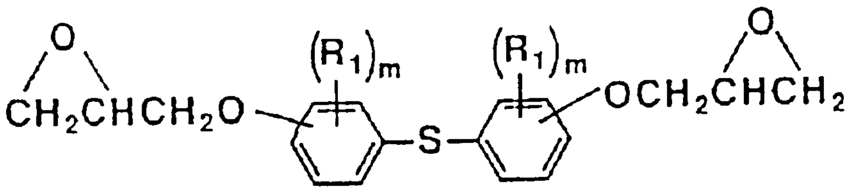

(1)

wherein $R_1$ represents a hydrogen atom, an alkyl or cycloalkyl group of 1 to 6 carbon atoms, a substituted or unsubstituted phenyl group or a halogen atom and when two or more $R_1$ are present in the same or different rings, they may be the same or different and m represents an integer of 0 to 4, and a polyhydric phenol as a curing agent. The present invention further relates to a resin-encapsulated semiconductor device prepared by encapsulating a semiconductor element with said epoxy resin composition.

Examples of the substituent $R_1$ of the epoxy resin represented by the formula (1) are a methyl group, ethyl group, propyl group, butyl group, amyl group, hexyl group, cyclohexyl group, phenyl group, tolyl group, xylyl group (including isomers), chlorine atom and bromine atom.

Furthermore, the epoxy resin used in the present invention can be obtained by the known process of glycidyl etherification of phenols. That is, this process comprises allowing a phenol to react with an epihalohydrin in the presence of an alkali such as sodium hydroxide. Especially, for obtaining high purity products, it is suitable to effect the reaction in an aprotic solvent as described in JP-A-60-31517, or in an ether compound as described in JP-A-62-34330.

The phenols used here are compounds represented by the following formula (2):

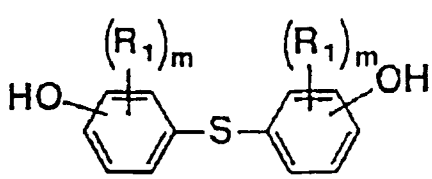

(2)

wherein $R_1$ and m are as defined in the formula (1). Examples of the phenols are enumerated below.

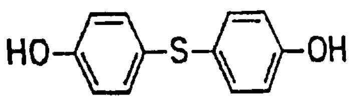

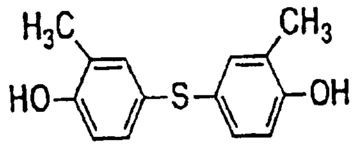

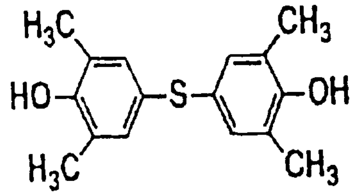

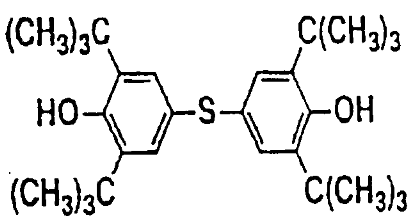

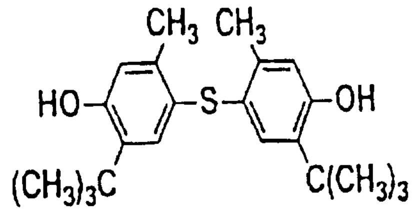

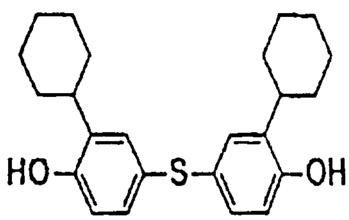

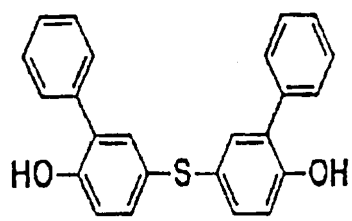

Furthermore, these compounds may have at least one halogen atom in their benzene ring.

As the curing agent used in the present invention, polyhydric phenols such as phenolic novolak are used.

Specific examples of the polyhydric phenols are polycondensates (so-called phenolic novolaks) of one or more phenols such as phenol, various alkylphenols and naphthol with aldehydes such as formaldehyde, acetaldehyde, acrolein, glyoxal, benzaldehyde, napthaldehyde and hydroxybenzaldehyde or ketones such as cyclohexanone and acetophenone; vinyl polymerization type polyhydric phenols such as polyvinylphenol and polyisopropenylphenol; and Friedel-Crafts reaction products of phenols with diols such as a compound represented by the following formula (4), dialkoxys such as a compound represented by the following formula (5) or dihalogens such as a compound represented by the following formula (6) and Friedel-Crafts reaction products of phenols with diolefins such as dicyclopentadiene and diisopropenylbenzene. Among them, phenolic novolaks are especially preferred from the points of workability and curability.

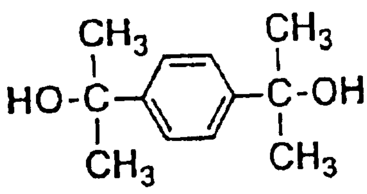

( 4 )

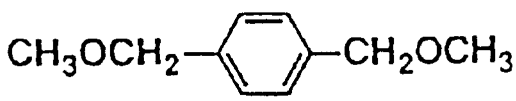

(5)

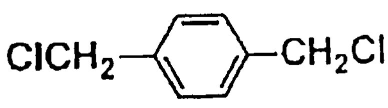

(6)

These curing agents may be used each alone or in combination of two or more.

The amount of the curing agent is preferably 0.7 to 1.2 equivalent per epoxy group. If the amount is less than 0.7 equivalent per epoxy group or more than 1.2 equivalent, curing is insufficient.

When the resin composition of the present invention is cured, known curing accelerators may be used. Examples of these curing accelerators are organic phosphine compounds such as triphenylphosphine, tri-4-methylphenylphosphine, tri-4-methoxyphenylphosphine, tributylphosphine, trioctylphosphine and tri-2-cyanoethylphosphine, tertiary amines such as tributylamine, triethylamine, 1,8-diazabicyclo(5,4,0)undecene-7 and triamylamine, quaternary ammonium salts such as benzyltrimethylammonium chloride, benzyltrimethylammonium hydroxide and triethylammoniumtetraphenyl borate, and imidazoles. These are not limitative. Among them, organic phosphine compounds, 1,8-diazabicyclo(5,4,0)undecene-7 and imidazoles are preferred from the points of moisture resistance and curability, and triphenylphosphine is especially preferred. Furthermore, known additives such as fillers, flame retardants, releasing agents and surface treating agents can be added to the composition, if necessary.

The fillers include, for example, silica, alumina, titanium white, talc, clay and glass fiber. Silica and alumina are especially preferred. The fillers which differ in shape (sphere or fragment) or size can be mixed to increase filling amount. Amount of the fillers added when the composition is used for encapsulating of semiconductors is 25-95% by weight, preferably 60-90% by weight based on the total weight of the composition. If the amount is less than 25% by weight, the composition is inferior in moisture resistance and if it is more than 95% by weight, the composition has the problem in moldability.

The flame retardants include, for example, phosphorus compounds, brominated epoxy resins and antimony trioxide. The releasing agents include, for example, natural waxes, synthetic waxes, higher fatty acids and metal salts thereof. The surface treating agents include, for example, silane coupling agents. Moreover, various elastomers may be added or may be previously allowed to react with the composition for reduction of stress. Examples of the elastomers are addition type or reaction type elastomers such as polybutadiene, butadiene-acrylonitrile copolymer, silicone rubber and silicone oil.

Semiconductor devices which can be encapsulated with the epoxy resin composition of the present invention include, for example, those of SOP type, SOJ type and QFP type.

For making resin-encapsulated semiconductor devices by encapsulating semiconductor elements with the epoxy resin compositions of the present invention, known molding methods such as transfer molding, compression molding and injection molding can be employed to perform curing and molding. Desirably, the molding is carried out at 150-180 °C for 30-180 seconds and postcuring is carried out at 150-180 °C for 2-16 hours.

The epoxy resin composition of the present invention has lower moisture absorption and is balanced in adhesiveness as encapsulating materials especially for electronic parts. Furthermore, resin-encapsulated semiconductor devices made using the composition are excellent in crack resistance in soldering.

Since the composition has lower viscosity than glycidyl ether of o-cresol novolak, fillers can be added in a large amount and moisture absorption is improved and reliability of the resulting articles can be enhanced.

The following nonlimiting examples illustrate the present invention.

In the examples, the "epoxy equivalent" is defined to be a molecular weight of epoxy resin per one epoxy group. The "hydrolyzable chlorine" is defined to be as follows: The epoxy resin is dissolved in dioxane and an alcoholic solution of potassium hydroxide is added thereto. The mixture is heated for 30 minutes under refluxing and chlorine ion released is subjected to back titration with an aqueous silver nitrate solution and the concentration in the compound is expressed by ppm.

Measurement of properties of resin and evaluation of kneaded product and cure molded products are conducted in the following manner.

Melt viscosity of resin: The melt viscosity is measured at 110°C and 150°C by a cone plate type viscometer (CONTRAVES Reomat 115).

Barcol hardness: This is measured in accordance with ASTM D-648 using 935 type hardness tester under conditions of 175°C/90 sec.

Glass transition temperature: This is measured by a thermo-mechanical analyzer (SHIMADZU DT-30).

Flexural strength and flexural modulus: These are measured in accordance with JIS K-6911 using a tensile tester (SHIMADZU IS-10T).

Water absorption: Change in weight is measured using a thermo-hygrostat (Advantec Toyo AGX-326) under the conditions of 85°C/85%RH.

Spiral flow: Evaluation is conducted in accordance with EMMI-1-66 under the conditions of 175°C/70 $kg/cm^2$.

Aluminum or copper peel strength (adhesion): The composition is transfer molded on an aluminum foil or a copper foil and the adhesion is evaluated by peel strength of the foil.

Crack resistance in soldering: A trial IC (QFP of 52 pins; thickness of package 2.05 mm) is allowed to absorb moisture under the conditions of 85°C/85%RH/72 hours and immediately thereafter, dipped in a soldering bath of 240°C for 30 seconds and the crack resistance is evaluated by the number of IC in which cracks have occurred. The number of the test IC is 20.

Reference Example 1

4,4'-Thiodiphenol (manufactured by Sumitomo Seika Co., Ltd., 109.0 g) was charged in a reaction vessel equipped with a thermometer, a stirrer, a dropping funnel and a condenser with a Dean Stark trap and dissolved in epichlorohydrin (647.5 g) and dimethyl sulfoxide (323.8 g). With keeping the reaction system under 42 torr, 48.6% sodium hydroxide (82.3 g) was continuously added dropwise at 48°C over a period of 5 hours.

Under the temperature of 48°C, the reaction was allowed to proceed with cooling and liquefying the azoetropic epichlorohydrin and water and returning the organic layer to the reaction system.

After completion of the reaction, unreacted epichlorohydrin was removed by concentration under reduced pressure and glycidyl ether containing by-produced salts and dimethyl sulfoxide was dissolved in methyl isobutyl ketone and the by-produced salts and dimethyl sulfoxide were removed by washing with water.

Epoxy equivalent weight and content of hydrolyzable chlorine of the resulting glycidyl ether were 172.1 g/equivalent and 345 ppm, respectively.

Reference Example 2

4,4'-Thiodi(2-methylphenol) (manufactured by Sumitomo Seika Co., Ltd., 123.0 g) was charged in a reaction vessel equipped with a thermometer, a stirrer, a dropping funnel and a condenser with a Dean Stark trap and dissolved in epichlorohydrin (647.5 g) and dimethyl sulfoxide (323.8 g). With keeping the reaction system under 42 torr, 48.6% sodium hydroxide (82.3 g) was continuously added dropwise at 48°C over a period of 5 hours.

Under the temperature of 48°C, the reaction was allowed to proceed with cooling and liquefying the azoetropic epichlorohydrin and water and with returning the organic layer to the reaction system.

Thereafter, the same procedure as in Reference Example 1 was conducted to obtain glycidyl ether. Epoxy equivalent weight and content of hydrolyzable chlorine were 186.1 g/equivalent and 310 ppm, respectively.

Reference Example 3

4,4'-Thiodi(2,6-dimethylphenol) (manufactured by Sumitomo Seika Co., Ltd., 137.0 g) was charged in a reaction vessel equipped with a thermometer, a stirrer, a dropping funnel and a condenser with a Dean Stark trap and dissolved in epichlorohydrin (647.5 g) and dimethyl sulfoxide (323.8 g). With keeping the reaction system under 42 torr, 48.6% sodium hydroxide (82.3 g) was continuously added dropwise at 48°C over a period of 5 hours.

Under the temperature of 48°C, the reaction was allowed to proceed with cooling and liquefying the azoetropic epichlorohydrin and water and with returning the organic layer to the reaction system.

Thereafter, the same procedure as in Reference Example 1 was conducted to obtain glycidyl ether. Epoxy equivalent weight and content of hydrolyzable chlorine were 198.8 g/equivalent and 150 ppm, respectively.

Reference Example 4

4,4'-Thiodi(3-methyl-6-t-butylphenol) (SUMILIZER WX-R manufactured by Sumitomo Chemical Co., Ltd., 179.0 g) was charged in a reaction vessel equipped with a thermometer, a stirrer, a dropping funnel and a condenser with a Dean Stark trap and dissolved in epichlorohydrin (647.5 g) and dimethyl sulfoxide (323.8 g). With keeping the reaction system under 44 torr, 48.5% sodium hydroxide (82.5 g) was continuously added dropwise at 48°C over a period of 5 hours.

Under the temperature of 48°C, the reaction was allowed to proceed with cooling and liquefying the azoetropic epichlorohydrin and water and with returning the organic layer to the reaction system.

Thereafter, the same procedure as in Reference Example 1 was conducted to obtain glycidyl ether. Epoxy equivalent weight and content of hydrolyzable chlorine were 244.4 g/equivalent and 180 ppm, respectively.

Reference Example 5

4,4'-Thiodi(2-methyl-6-t-butylphenol) (manufactured by Sumitomo Seika Co., Ltd., 179.0 g) was charged in a reaction vessel equipped with a thermometer, a stirrer, a dropping funnel and a condenser with a Dean Stark trap and dissolved in epichlorohydrin (647.5 g) and dimethyl sulfoxide (323.8 g). With keeping the reaction system under 44 torr, 48.5% sodium hydroxide (82.5 g) was continuously added dropwise at 48°C over a period of 5 hours.

Under the temperature of 48°C, the reaction was allowed to proceed with cooling and liquefying the azoetropic epichlorohydrin and water and with returning the organic layer to the reaction system.

Thereafter, the same procedure as in Reference Example 1 was conducted to obtain glycidyl ether. Epoxy equivalent weight and content of hydrolyzable chlorine were 242.0 g/equivalent and 90 ppm, respectively.

Examples 1-6 and Comparative Examples 1-2

A glycidyl ether, a phenol novolak (TAMANOL 785 manufactured by Arakawa Chemical Industry Co., Ltd.), triphenyl phosphine as a curing accelerator, fused silica (FS-891 manufactured by Denki Kagaku Kogyo K.K.) and a spherical silica (FB-74 manufactured by Denki Kagaku Kogyo K.K.) as filler, carnauba wax as a releasing agent and a coupling agent (SH-6040 manufactured by Toray Dow Corning Silicone Co., Ltd.) in the amounts (g) as shown in Table 1 were blended and kneaded with heating by a roll and then transfer molded. The glycidyl ethers used in Examples 1-6 were those obtained in Reference Examples and the glycidyl ether used in Comparative Examples was glycidyl ether of o-cresol novolak (SUMI® EPOXY ESCN-195 manufactured by Sumitomo Chemical Co., Ltd.).

The molded products were further subjected to postcure in an oven at 180° C for 5 hours to obtain cure molded products. Glass transition temperature, water absorption, flexural strength and flexural modulus of the products were measured. The results are shown in Table 1.

Table 1

| | | Example 1 | Example 2 | Example 3 | Example 4 |
|---|---|---|---|---|---|
| Glycidyl ether | | Reference Example 1 | Reference Example 2 | Reference Example 3 | ← |
| Melt viscosity of glycidyl ether (poise) | 110°C | 0.13 | 0.11 | 0.24 | ← |
| | 150°C | 0.05 | 0.05 | 0.08 | ← |
| Amount of glycidyl ether | | 100 | ← | ← | ← |
| Phenolic novolak | | 61.6 | 57.0 | 53.3 | ← |
| Triphenyl phosphine | | 1.5 | ← | ← | ← |
| Spherical silica | | 404.8 | 397.7 | 388.4 | 598.8 |
| Fused silica | | 102.3 | 99.4 | 97.1 | 149.7 |
| Releasing agent | | 1.5 | ← | ← | ← |
| Coupling agent | | 2.0 | ← | ← | ← |
| Spiral flow (INCH) | | 45.5 | 58.0 | 59.5 | 31.0 |
| Barcol hardness | | 56 | 45 | 40 | 60 |
| Glass transition temperature (°C) | | 125 | 115 | 128 | 129 |
| Flexural strength ($kg/mm^2$) | | 14.5 | 16.5 | 13.5 | 15.0 |
| Flexural modulus ($kg/mm^2$) | | 1550 | 1620 | 1630 | 1980 |
| Water absorption (%) | 24H | 0.16 | 0.13 | 0.14 | 0.10 |
| | 72H | 0.28 | 0.22 | 0.23 | 0.16 |
| Aluminium peel strength (g/cm) | | 490 | 770 | 770 | 720 |
| Copper peel strength (g/cm) | | 360 | 595 | 560 | 510 |
| Crack resistance in soldering | | 6 | 7 | 4 | 1 |

Note: The symbol " ← " means that the value is the same as in the left column.

Table 1 (continued)

| | | Example 5 | Example 6 | Comparative Example 1 | Comparative Example 2 |
|---|---|---|---|---|---|
| Glycidyl ether | | Reference Example 4 | Reference Example 5 | ESCN-195 | ← |
| Melt viscosity of glycidyl ether (poise) | 110°C | – | – | – | – |
| | 150°C | 0.10 | 0.10 | 5.0 | ← |
| Amount of glycidyl ether | | 100 | ← | ← | ← |
| Phenolic novolak | | 43.4 | 43.8 | 54.0 | ← |
| Triphenyl phosphine | | 1.5 | ← | ← | ← |
| Spherical silica | | 363.3 | 384.3 | 390.1 | 602.7 |
| Fused silica | | 90.8 | 91.1 | 97.5 | 150.7 |
| Releasing agent | | 1.5 | ← | ← | ← |
| Coupling agent | | 2.0 | ← | ← | ← |
| Spiral flow (INCH) | | 61.1 | 55.0 | 44.0 | 20.0 |
| Barcol hardness | | 25 | 18 | 74 | 82 |
| Glass transition temperature (°C) | | 120 | 110 | 165 | 165 |
| Flexural strength ($kg/mm^2$) | | 13.4 | 13.1 | 15.5 | 16.7 |
| Flexural modulus ($kg/mm^2$) | | 1620 | 1840 | 1660 | 2030 |
| Water absorption (%) | 24H | 0.11 | 0.10 | 0.16 | 0.13 |
| | 72H | 0.18 | 0.17 | 0.29 | 0.23 |
| Aluminium peel strength (g/cm) | | 750 | 720 | 340 | 280 |
| Copper peel strength (g/cm) | | 450 | 700 | 140 | 90 |
| Crack resistance in soldering | | 3 | 3 | 20 | 17 |

Note: The symbol " ← " means that the value is the same as in the left column.

## Claims

1. An epoxy resin composition comprising as essential components an epoxy resin represented by the following formula (1):

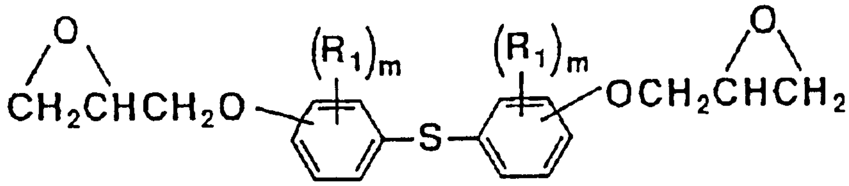

(1)

wherein $R_1$ represents a halogen atom, an alkyl or cycloalkyl group of 1 to 6 carbon atoms, a substituted or unsubstituted phenyl group or a halogen atom and when two or more $R_1$ are present in the same or different rings, they may be the same or different and m represents an integer of 0 to 4, and a polyhydric phenol as a curing agent.

**2.** A resin-encapsulated semiconductor device produced by encapsulating a semiconductor element with the epoxy resin composition of claim 1.

European Patent Office

# EUROPEAN SEARCH REPORT

Application Number
EP 93 11 8081

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A | FR-A-1 541 474 (THE DOW CHEMICAL COMPANY)<br>* examples 6,7 * | 1 | C08G59/30<br>C08G59/62<br>H01L23/29<br>C07D303/23 |
| A | GB-A-1 170 105 (THE DOW CHEMICAL COMPANY)<br>* claims * | 1 | |
| A | FR-A-2 093 785 (THE DOW CHEMICAL COMPANY)<br>* claims 1,6,7 * | 1 | |
| A | EP-A-0 442 568 (SHELL)<br>* claim 1 * | 1 | |

TECHNICAL FIELDS SEARCHED (Int.Cl.5)
C08G
C07D

The present search report has been drawn up for all claims

| Place of search | Date of completion of the search | Examiner |
|---|---|---|
| THE HAGUE | 25 January 1994 | Deraedt, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document